# EUROPEAN PATENT APPLICATION

(11) **EP 4 201 410 A1**
(43) Date of publication of application: **28.06.2023**
(21) Application number: 21216699.5
(22) Date of filing: 21.12.2021
(51) Int. Cl.: A61K 31/714, A61P 17/16, A61P 37/00, A61Q 17/04

(54) **NOVEL USE OF VITAMIN B12**

(71) Applicant: DSM IP Assets B.V., 6411 TE Heerlen (NL)
(72) Inventor: IMFELD, Dominik, 4303 Kaiseraugst (CH)
(74) Representative: Berg, Katja

(57) **Abstract**

The present invention relates to the use of Vitamin B12 as mast cell degranulation inhibitor as well as to the prevention or treatment of ailments in which mast cell degranulation contributes to the pathology, and/or symptoms, and/or progression, of said ailments.

## Description

The present invention relates to the use of vitamin B12 as mast cell degranulation inhibitor as well as to the prevention or treatment of ailments in which mast cell degranulation contributes to the pathology, and/or symptoms, and/or progression, of said ailments.

Mast cells (MCs) are immune cells which are present in nearly all tissues but are prominent in organs that are exposed to the environment such as the skin. MCs cause allergic symptoms by releasing huge variety of soluble preformed or newly-synthesised mediators, including proteases, histamine, lipid mediators, cytokines and chemokines. This release occurs when the allergy antibody IgE, which is present on the mast cell surfaces, binds to proteins that cause allergies, called allergens. This triggering is called activation, and the release of these mediators is called degranulation. MC degranulation appears to be inducible by environmental factors including ultraviolet radiation (UVR), environmental estrogens or combustion products, such as diesel exhaust particles. Degranulation and subsequent mediator release of skin MC is responsible for the appearance of skin allergy reactions such as edema or hives.

Thus, there is an ongoing need for cosmetically acceptable ingredients which act as mast cell degranulation inhibitor and can thus be used to counteract skin conditions associated therewith and thus to ameliorate the health and appearance of the skin.

Surprisingly it has now been found that vitamin B12 efficiently inhibits mast cell degranulation

Thus, in a first embodiment, the present invention relates to a composition comprising an effective amount of vitamin B12 for its use in a dermatological treatment as agent for protecting skin cells against mast cell degranulation, in particular mast cell degranulation caused by environmental factors such as in particular by ultraviolet radiation and/ or air pollutants.

In another embodiment, the present invention concerns a cosmetic or dermatological composition comprising an effective amount of vitamin B12 for use in the prevention or treatment of skin conditions caused by environmental factors, in particular UV-radiation (UV-R) and/ or air pollution-induced mast cell degranulation and optionally appreciating the effect.

In a further embodiment, the present invention relates to a method to prevent or treat skin conditions caused by, preferably UV-radiation and/ or air pollution-induced induced, mast cell degranulation comprising the following steps:
a) providing a cosmetic or dermatological composition comprising vitamin B12, and
b) applying an amount of said composition to human skin or scalp that is sufficient to reduce or inhibit mast cell degranulation.

The inhibitory effect of vitamin B12 on mast cell degranulation according to the present invention is understood to reduce or inhibit the release of histamine and in turn allergic skin reactions respectively symptoms resulting thereof.

In a further aspect, the present invention relates to vitamin B12 (and/or cosmetic or dermatological compositions comprising vitamin B12) for: the use in the prevention and/ or treatment of a skin condition associated with mast cell degranulation; in the treatment of a skin condition in which the mast cell degranulation contributes to the pathology, and/or symptoms, and/or progression, of said skin condition, in inhibiting mast cell degranulation (including in a subject in need thereof), and/or as a mast cell degranulation inhibitor. Specific skin conditions are e.g., irritated skin, sensitive skin, skin erythema, and skin ageing.

In another aspect, there is provided a use of vitamin B12 (and/or cosmetic or dermatological compositions comprising vitamin B12): in the treatment of a disease or disorder associated with mast cell degranulation; in the treatment of a disease or disorder in which the mast cell degranulation contributes to the pathology, and/or symptoms, and/or progression, of said disease/disorder; in inhibiting mast cell degranulation (including in a subject in need thereof), and/or as an mast cell degranulation inhibitor.

In another aspect, there is provided a use of vitamin B12 (and/or cosmetic or dermatological compositions comprising Vitamin B12) in the manufacture of a medicament for: the treatment of a disease or disorder associated with mast cell degranulation; the treatment of a disease or disorder in which the mast cell degranulation contributes to the pathology, and/or symptoms, and/or progression, of said disease/disorder, and/or inhibiting mast cell degranulation (including in a subject in need thereof).

In another aspect, there is provided a method of treating a disease or disorder in which mast cell degranulation contributes to the pathology, and/or symptoms, and/or progression, of said disease/disorder, comprising administering an effective amount of vitamin B12, for instance to a subject (in need thereof).

The present invention also relates to vitamin B12 as mast cell degranulation inhibitor.

In all embodiments of the present invention, preferably, vitamin B12 is used in the prevention and/ or treatment of allergic skin reactions respectively symptoms such as hive or erythema.

The term 'skin' as used in this document, is meant to include the external surface of mammals, especially humans and includes the skin and the scalp. Preferred skin in all embodiments of the present invention is facial and body skin such as most preferably facial skin.

The term 'prevention' as used herein refers to lessening the risk of developing a skin condition associated with mast cell degranulation such as scarring.

The term 'treatment' as used herein refers to an amelioration of symptoms, delaying the onset and/ or reduction of the duration of any diseases associated with mast cell degranulation. The treatment can be prophylactic (cosmetic) or therapeutic. Preferably, the treatment is prophylactic.

The term 'mast cell degranulation inhibitor' refers to a compound capable of reducing respectively inhibiting histamine release caused by degranulation of mast cells. The mast cell degranulation may be stimulated by allergens through cross-linking with immunoglobulin E receptors, physical injury through pattern recognition receptors for damage-associated molecular patterns, microbial pathogens through pattern recognition receptors for pathogen-associated molecular patterns, and various compounds through their associated G-protein coupled receptors or ligand-gated ion channels. More particularly, vitamin B12 is capable of reducing, respectively inhibiting the release of histamine.

The term 'effective amount' as used herein refers to an amount necessary to obtain the physiological effect. The physiological effect may be achieved by one application dose or by repeated applications. The dosage administered may, of course, vary depending upon known factors, such as the physiological characteristics of the particular cosmetic or dermatological composition comprising vitamin B12 and its mode and route of administration; the age, the nature and extent of the symptoms; the kind of concurrent treatment; the frequency of treatment; and the effect desired and can be adjusted by a person skilled in the art.

The term 'dermatological' as used herein may refer to cosmetic (non-therapeutic) as well as pharmaceutical (therapeutic) treatments. In all embodiments of the present invention cosmetic treatments i.e. treatments intended for beautifying the skin are preferred.

The term 'cosmetic or dermatological composition' as used herein refers to compositions, which are used to treat, care for or improve the appearance of the skin and/or the scalp. Particular advantageous cosmetic or dermatological compositions are skin care preparations.

Environmental air pollutants according to the present invention are in dust particles such as diesel dust, polycyclic aromatic hydrocarbons (PAHs) or estrogenic environmental pollutants (environmental estrogens), such as e.g. polychlorinated biphenyls (PCBs), and phytoestrogens such as isoflavones or lignans.

Vitamin B12 (INCI cyanocobalamin) is e.g. commercially available at DSM nutritional Products Ltd. as Quali^{®}-B.

In all embodiments of the present invention preferably the cells are skin cells such as in particular epithelial cells, especially keratinocytes, melanocytes, fibroblasts or dendritic cells.

In all embodiments of the present invention preferably the treatment is non-therapeutic, i.e. cosmetic.

In all embodiments of the present invention, preferably, the amount of vitamin B12 in the compositions according to the present invention is selected in the range of 0.0001 wt.-% to 0.1 wt.-%, preferably in the range of 0.001 wt.-% to 0.05 wt.-%, most preferably in the range of 0.001 to 0.025 wt.-%, based on the total weight of the composition. Further suitable ranges include 0.001 wt.% to 0.01 wt.-%.

Preferably, the amount of the cosmetic or dermatological composition according to the present invention to be applied to the skin is selected in the range of 0.1 to 3 mg/ cm² skin, such as preferably in the range of 0.1 to 2 mg/ cm² skin and most preferably in the range of 0.5 to 2 mg / cm² skin.

Preferably, in all embodiments of the present invention, vitamin B12 is suitable for use in inhibiting the effects of UV-R, preferably UV-B, exposure, or alleviating the effects of UV-R, preferably UV-B, induced damage on the skin caused by mast cell degranulation.

Thus, preferably, in all embodiments of the present invention, the compositions according to the present invention, next to vitamin B12, further comprises at least one UV-R, preferably UV-A filter substance, most preferably extending protection in the visible light range.

Suitable UV filter substances absorbing UV-radiation such as in particular UV-A and/ or UV-B radiation are well known to a person skilled in the art and encompass oil soluble, water soluble and inorganic UV-filter substances.

Advantageous oil soluble UV-filter substances according to the present invention encompass dibenzoylmethane derivatives such as butyl methoxydibenzoylmethane (e.g. commercially available as PARSOL^{®} 1789 at DSM Nutritional Products Ltd), organopolysiloxanes functionalized with at least one UV-light absorbing group such as polysilicone-15 (e.g. commercially available as PARSOL^{®} SLX at DSM Nutritional Products Ltd), diphenylacrylates such as octocrylene (e.g. commercially available as PARSOL^{®} 340 at DSM Nutritional Products Ltd), cinnamates such as octyl methoxycinnamate (e.g. commercially available as PARSOL^{®} MCX at DSM Nutritional Products Ltd), triazine derivatives such as Bis-Ethylhexyloxyphenol Methoxyphenyl Triazine (commercially available as PARSOL^{®} SHIELD at DSM Nutritional Products Ltd), Ethyl Hexyl Triazone (e.g. commercially available as PARSOL^{®} EHT at DSM Nutritional Products Ltd), Diethylhexyl butamido Triazone (Uvasorb^{®} HEB), Diethylamino Hydroxybenzoyl Hexyl Benzoate (Uvinul^{™} A+), salicylates such as octylsalicylate (ethylhexyl salicylate) and homosalate (e.g. commercially available as PARSOL EHS, respectively HMS EHT at DSM Nutritional Products Ltd) as well as mixtures thereof.

The total amount of the oil soluble UV-filter substance(s) in the composition according to the invention is preferably selected in the range of about 0.1 to 25 wt.-%, more preferably in the range of about 0.5 to 20 wt.-%, most preferably in the range of 1 to 15 wt.-% based on the total weight of the composition.

Advantageous water soluble UV-filter substance according to the present invention encompass 2-phenylbenzimidazol-sulphonic acid or 2,2'-(1,4-phenylene)bis-1H-benzimidazole-5,7-disulfonic acid as well as the respective salts thereof. Preferably the water soluble UV-filter substance is 2-phenylbenzimidazol-sulphonic acid (e.g. commercially available as PARSOL^{®} HS at DSM Nutritional Products Ltd).

The total amount of the water soluble UV-filter substance(s) in the composition according to the invention is preferably selected in the range of about 0.1 to 5 wt.-%, more preferably in the range of about 0.5 to 4 wt.-%, most preferably in the range of 1 to 3 wt.-% based on the total weight of the composition

Advantageous inorganic UV-filter substances according to the present invention encompass UV-light absorbing pigments such as e.g. microparticulated Zink oxide or Titanium dioxide. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art. In all embodiment of the present invention, preferably the inorganic UV-filter substance is selected from titanium dioxide having an inner silica and an outer dimethicone coating which is commercially available as PARSOL^{®} TX at DSM Nutritional Products Ltd or from zinc oxide having a triethoxycaprilylsilane coating which is commercially available as PARSOL^{®} ZX at DSM Nutritional Products Ltd.

The total amount of the inorganic UV-filter substance(s) in the composition according to the invention is preferably selected in the range of about 0.1 to 10 wt.-%, more preferably in the range of about 0.5 to 8 wt.-%, most preferably in the range of 2 to 5 wt.-% based on the total weight of the composition.

In all embodiments of the present invention, preferably, the composition is applied on the skin before or after exposure or contact with ultraviolet radiation and/ or air pollutants.

The cosmetic or dermatological compositions according to the invention are intended for topical application, which is to be understood as the external application to keratinous substances, such as in particular the skin.

As the cosmetic or dermatological compositions according to the invention are intended for topical application, they comprise a physiologically acceptable medium, that is to say a medium compatible with keratinous substances, such as in particular the skin. In particular the physiologically acceptable medium is a cosmetically, respectably dermatologically acceptable carrier.

The term 'cosmetically acceptable carrier' respectively 'dermatologically acceptable carrier' as used herein refers to a physiologically acceptable medium which is compatible with keratinous substances. Suitable carriers are well known in the art and are selected based on the end-use application. Preferably, the carriers of the present invention are suitable for application to skin (e.g., sunscreens, creams, milks, lotions, masks, serums, hydrodispersions, foundations, creams, creamgels, or gels etc.). Such carriers are well-known to one of ordinary skill in the art and can include one or more compatible liquid or solid filler diluent, excipient, additive or vehicle which are suitable for application to skin. The exact amount of carrier will depend upon the level of the active(s), respectively active blends and any other optional ingredients that one of ordinary skill in the art would classify as distinct from the carrier (e.g., other active components). The cosmetic compositions of the present invention preferably comprise from about 70% to about 99.999%, more preferably from about 85% to about 99.99%, still more preferably from 90% to about 99%, and most preferably, from about 93% to about 98%, by weight of the cosmetic composition, of a carrier.

The cosmetic compositions of the present invention can be formulated into a wide variety of product types, including creams, waxes, pastes, lotions, milks, mousses, gels, oils, tonics, and sprays. Preferably the active(s), respectively the active blends are formulated into lotions, creams, gels, and tonics. These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art.

If cosmetic or dermatological compositions of the present invention are formulated as an aerosol and applied to the skin as a spray-on product, a propellant is added to the cosmetic composition.

The cosmetic or dermatological compositions according to the present invention can be prepared by conventional methods in the art such as e.g. by admixing an active or an active blend according to the present with the cosmetically acceptable carrier.

The cosmetic or dermatological compositions of the invention (including the carrier) may comprise further conventional adjuvants and additives, such as preservatives/antioxidants, fatty substances/oils, water, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such cosmetic or dermatological compositions.

In accordance with the present invention, the cosmetic or dermatological compositions according to the invention may also comprise further cosmetically active ingredients conventionally used in cosmetic and/ or dermatological compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic or dermatological excipients, diluents, adjuvants, additives as well as active ingredients commonly used in the skin care industry which are suitable for use in the cosmetic or dermatological compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of further active ingredients as well as the excipients, diluents, adjuvants, additives etc. can, based on the desired product form and application, easily be determined by the skilled person. The additional ingredients can either be added to the oily phase, the aqueous phase or separately as deemed appropriate.

The further cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic or dermatological compositions according to the present invention may be in the form of a suspension or dispersion in solvents or fatty substances, or alternatively in the form of an emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the cosmetic or dermatological composition is an emulsion, such as in particular an O/W, W/O, Si/W, W/Si, O/W/O, W/O/W multiple or a pickering emulsion, then the amount of the oily phase present in such cosmetic or dermatological emulsions is preferably at least 10 wt.-%, such as in the range of 10 to 60 wt.-%, preferably in the range of 15 to 50 wt.-%, most preferably in the range of 15 to 40 wt.-%, based on the total weight of the cosmetic or dermatological composition.

In one embodiment, the cosmetic or dermatological compositions according to the present invention are advantageously in the form of an oil-in-water (O/W) emulsion comprising an oily phase dispersed in an aqueous phase in the presence of an O/Wemulsifier. The preparation of such O/W emulsions is well known to a person skilled in the art.

If the cosmetic or dermatological composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol^{®} A from DSM Nutritional Products Ltd.), diethanolamine cetyl phosphate (e.g. as Amphisol^{®} DEA from DSM Nutritional Products Ltd.), potassium cetyl phosphate (e.g. as Amphisol^{®} K from DSM Nutritional Products Ltd.), sodium cetearylsulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C 10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the cosmetic or dermatological composition.

Particular suitable O/W emulsifiers to be used in the cosmetic or dermatological compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

A particular suitable O/W emulsifier to be used in the cosmetic or dermatological compositions according to the invention is potassium cetyl phosphate e.g. commercially available as Amphisol^{®} K at DSM Nutritional Products Ltd Kaiseraugst.

Another particular suitable class of O/W emulsifiers are non-ionic self-emulsifying systems derived from olive oil e.g. known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to cosmetic or dermatological compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%.

The cosmetic or dermatological compositions according to the invention in general have a pH in the range of 3 to 10, preferably a pH in the range of 4 to 8 and most preferably a pH in the range of 4 to 7.5. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The following examples are provided to further illustrate the effects of the present invention. These examples are illustrative only and are not intended to limit the scope of the invention in any way.

### Examples

This experiment was designed to evaluate the mast cell degranulation efficacy of vitamin B12. Mast cells were used as the testing model, and compound C48/80* was used to stimulate mast cells into a sensitive status. The histamine was released after the degranulation of mast cells, so the degranulation rate can reflect histamine release level. A soothing effect of test compound administered can also be evaluated by the degranulation rate of mast cells.

*Compound 48/80 is a polymer produced by the condensation of N-methyl-p-methoxyphenethylamine with formaldehyde. It promotes histamine release, and in biochemical research, compound 48/80 is used to promote mast cell degranulation.

C48/80 can bypass the traditional receptor and directly activate the trimeric small molecule G protein (GTPase). The βγ subunit of the trimeric small molecule G protein activates Dbl family of G protein, such as Rho, Rac, Cdc42. The other is that it activates PLCβ in a direct or indirect way, thereby activating PKC and opening intracellular calcium stores, triggering exocytosis. In addition to the release of various inflammatory factors and inflammatory mediators after mast cell activation, the release of histamine is the main factor for mast cell activation and exerts pathophysiological effects among others triggering allergic reactions.

### Experimental procedure:

Mast cells (P815) were incubated in six well plates for 24hours then test sample was added and incubated for 60min, then C48/80 was added at 50mg/ml and incubated for another 60min. The reaction was stopped in the ice bath, then cell morphology was assessed by microscopy using IPP software to quantify degranulation.

| Group | Mean | SD | P-value |
|---|---|---|---|
| Blank Control | 0.00% | 0.00% | / |
| Negative Control | 94.81 % | 0.24% | 0.000## |
| Positive Control (cromolyn sodium 1mg/ml) | 54.11 % | 4.43% | 0.000** |
| Vitamin B12 0.1% | 69.10% | 2.90% | 0.000** |

Note: When T-test was used for statistical analysis, Negative Control group was compared with Blank Control group, the significance was represented by ## with p-value<0.01. Positive Control group and sample group was compared with Negative Control group, the significance was represented by **, p-value <0.01.

Compared with the Blank Control group, the degranulation rate of Negative Control group was significantly increased (P<0.01) after C48/80 stimulation, and the cells showed obvious degranulation morphology, indicating the success of the experimental modeling.

Compared with the Negative Control group, the degranulation rate of Positive Control group was significantly decreased (P<0.01) after adding cromolyn sodium, and the number of degranulated cells in the field of vision was significantly reduced, indicating that the experimental system was effective.

Compared with the Negative Control group, Vitamin B12 0.1% could significantly inhibit (P<0.01) mast cell degranulation at the concentration of 0.15%.

## Claims

1. A composition comprising an effective amount of vitamin B12 for its use in a dermatological treatment as agent for protecting skin cells against mast cell degranulation,

2. The composition according to claim 1, wherein vitamin B12 is used as agent for the prevention or treatment of an, preferably local, allergic-skin reaction.

3. The composition according to claim 1 and/ or 2, wherein the mast cell degranulation is caused by environmental factors, preferably by ultraviolet radiation and/ or air pollutants, most preferably by UVB radiation.

4. The composition according to anyone or more of claims 1 to 3, wherein the composition is applied on the skin before or after exposure or contact with ultraviolet radiation and/ or air pollutants.

5. The composition according to anyone or more of claims 1 to 4, wherein the effective amount is selected in the range of 0.0001 wt.-% to 0.1 wt.-%, preferably in the range of 0.001 wt.-% to 0.05 wt.-%, most preferably in the range of 0.001 to 0.025 wt.-%, based on the total weight of the composition.

6. The composition according to anyone or more of claims 1 to 5, wherein the amount of the composition to be applied to the skin is selected in the range of 0.1 to 3 mg/ cm² skin.

7. The composition according to anyone or more of claims 1 to 5, wherein the amount of the composition to be applied to the skin is selected in the range of 0.1 to 2 mg/ cm² skin.

8. The composition according to anyone or more of claims 1 to 5, wherein the amount of the composition to be applied to the skin is selected in the range of 0.5 to 2 mg / cm² skin.

9. The composition according to claim 8, wherein the composition in addition comprises at least one UV filter substance.

10. The composition according to anyone or more of claims 1 to 9, wherein the composition is a skin care preparation.

11. The composition according to anyone or more of claims 1 to 10, wherein the cosmetic or dermatological composition is an O/W emulsion comprising an oily phase dispersed in an aqueous phase in the presence of an O/W emulsifier.

12. The composition according to claim 11, **characterized in that** the O/W emulsifier is potassium cetyl phosphate.

13. A method to prevent or treat skin conditions caused by mast cell degranulation comprising the following steps:
a) providing a topical composition comprising vitamin B12, and
b) applying an amount of said topical composition to human skin or scalp that is sufficient to reduce or inhibit mast cell degranulation.

14. A method of treating a disease or disorder in which mast cell degranulation contributes to the pathology, and/or symptoms, and/or progression, of said disease/disorder, said method comprising administering an effective amount of vitamin B12 to a subject in need thereof.

15. Vitamin B12 as mast cell degranulation inhibitor.
